(19) **Europäisches Patentamt**

**European Patent Office**

**Office européen des brevets**

(11) Veröffentlichungsnummer: **0 280 354 B1**

(12) **EUROPÄISCHE PATENTSCHRIFT**

(45) Veröffentlichungstag der Patentschrift: **17.06.92**

(21) Anmeldenummer: **88200215.7**

(22) Anmeldetag: **08.02.88**

(51) Int. Cl.⁵: **A61N 5/06**, C08G 18/18

(54) **Besonnungskombination.**

(30) Priorität: **12.02.87 NL 8700338**

(43) Veröffentlichungstag der Anmeldung:
**31.08.88 Patentblatt 88/35**

(45) Bekanntmachung des Hinweises auf die
Patenterteilung:
**17.06.92 Patentblatt 92/25**

(84) Benannte Vertragsstaaten:
**AT BE CH DE GB LI LU NL SE**

(56) Entgegenhaltungen:
**EP-A- 0 088 377**
**DE-A- 3 303 794**
**DE-A- 3 440 478**
**US-A- 3 862 150**

(73) Patentinhaber: **den Dekker, Gerardus Matthijs**
**Weberstraat 13**
**NL-5102 BJ Dongen (N.B.)(NL)**

(72) Erfinder: **den Dekker, Gerardus Matthijs**
**Weberstraat 13**
**NL-5102 BJ Dongen (N.B.)(NL)**

(74) Vertreter: **Siemens, Andreas Meinhard Ernest,**
**Dipl.-Ing.**
**SIEMENS & CIE. Roskam 8**
**NL-4813 GZ Breda(NL)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung bezieht sich auf eine Konstruktion einer Besonnungskombination zum Bestrahlen des gesamten Körpers mit ultravioletter Strahlung.

Künstliche Besonnungsvorrichtungen für diesen Zweck sind bereits seit einigen Jahren bekannt.

Sie bestehen aus einer Sonnenbank und einer Sonnenkuppel und sie sind an die Stelle getreten der bereits seit Dezennien bekannten Höhensonnen. Die Apparate für die Besonnungstherapie wurden erheblich verbessert durch die Anwendung von röhrenförmigen Gasentladungslampen deren Strahlung durch die Füllung und Absorbtionsschichten so ausgewählt werden konnte, dass derjenige Teil der Strahlung, welcher Verbrennung der Haut und Gefahr für die Augen erzeugen kann, nahezu völlig unterdrückt werden konnte.

Die ultravioletten Strahlen von derartigen Besonnungsvorrichtungen für den Menschen haben vor allem Aufmerksamkeit bekommen als Bräunungsmittel, also für die kosmetische Behandlung.

Sie sind jedoch auch für die allegemeine Gesundheitspflege von grosser Bedeutung, weil sie nicht nur die Pigmenterzeugung verursachen doch auch die Umsetzung von Vorprodukten wichtiger Vitamine in diese Vitamine fördern wie die Umsetzung von Ergosterol in das Vitamin $D_2$ (Ergocalciferol).

Ein Mangel an Sonne kann einen Mangel an Vitamin verursachen.

Dies kann durch eine UV-Vorrichtung ausgeglichen werden.

Die Strahlung von Gasentladungsröhren für künstliches Sonnenlicht kann in die Spektralgebiete wie in Tabelle I dargelegt eingeteilt werden:

Tabelle I:

| | | |
|---|---|---|
| UV-A | 300-400 nm | Umsetzung von Pigment. |
| UV-B | 200-300 nm | Pigmenterzeugung. |
| UV-C | 100-200 nm | verursacht Erythem. |

Die geeignetsten Gasentladungsröhren für diesen besonderen Zweck strahlen hauptsächlich UV-A mit einem geringen Anteil an UV-B ab, während UV-C durch eine mineralische Filterverkleidung innerhalb der Röhren ausgefiltert wird.

Die Anwendung dieser Röhren hat dadurch grosse Bedeutung erlangt. Es bestehen jedoch noch einige technische und physiologische Nachteile, die durch die Konstruktion gemäss der vorliegenden Erfindung aufgehoben werden.

Nach dem Stande der Technik werden die Röhren in der Sonnenbank und der Sonnenkuppel in einem Metallrahmen unterhalb beziehungsweise oberhalb einer metallischen Spiegelschicht montiert und mit einer Platte aus Polyakrylatkunststoff abgedeckt.

In der Patentanmeldung DE-A-3.303.794 ist eine Vorrichtung zur UV-Bestrahlung beschrieben, die aus einem Metallrahmen, einer Liege und einer darüber gestellten Kappe besteht, wovon die Kappe durch eine teleskopisch funktionierende Vorrichtung unterstützt wird, die besteht aus einem Zylinder mit Kolben wobei der Kolben durch eine drehbare Schraubenachse mittels eines Elektromotors mit Schalter aufwärts und abwärts bewegt werden kann.

Metallrahmen sind schwer, und das auf- und abschieben ist ziemlich umständlich.

Einstellung von Hand wäre sicherer und leichter.

In der Patentanmeldung DE-A-3.440.478 wird eine Vorrichtung für Bestrahlungszwecke beschrieben, deren für die Aufnahme der Bestrahlungsröhren bestimmter Teil z.B aus einem einstückigen, wannenförmigen Kunststoffhartschaumkörper besteht. Ein derartiger Körper wird entweder als Bestrahlungsliege oder als hängender Fluter verwendet. Eine etwaige mechanische Verbindung von zwei solchen Kunststoffhartschaumkörpern wird nicht erwähnt. Insbesondere ist dabei kein Stativ zur Positionierung mit der Hand vorgesehen. Die konstruktiven, ergonomischen und Sicherheitsprobleme wurden durch die Besonnungskombination gemäss der vorliegenden Erfindung gelöst, indem die Vorrichtung, welche aus einer Liege, deren beide Längskanten in einem Winkel von etwa 30° nach oben gebogen sind, und einem Oberteil (Sonnenkuppel), deren beide Längskanten in einem Winkel von etwa 30° nach unten gebogen sind, besteht, wobei beide Teile je etwa 10 UV-Gasentladungsröhren enthalten, die sich über die gesamte Länge erstrecken, dadurch gekennzeichnet ist, dass beide Teile der Kombination je aus einem nahtlosen Ganzen von aufgeschäumtem Hartpolyurethan bestehen, in welchen sämtliche Halterungs,- Leitungs-und Schaltteile deppelt isoliert eingebettet sind, und wobei die beiden genannten Teile an den Endkanten von Kopf- und Fussende mit einander verbunden sind durch eine Kippstativvorrichtung, die aus zwei zu einem Winkel gebogenen Profilen besteht, und deren Enden in drehbarer Lagerung jeweils die Endkanten des Liegeteils und des Oberteils erfassen, sodass einzig durch diese beiden Profile eine stabile Stellung in jeder gewünschten Position erreicht wird, und der Oberteil von Hand anstrengungslos aus und abwärts bewegt werden kann.

Durch diese beiden zusammenwirkenden Profile bleibt der Oberteil in jeder Position zwischen nahezu senkrecht und nachgerade waagerecht im Gleichgewicht stehen.

Die Erfindung umfaßt auch ein Verfahren zur Herstellung einer solchen Besonnungskombination

Das Material, aus dem die Körper der Liege und des Oberteils gefertigt sind, besteht aus einem ganzstückigen nahtlos in einer Form mit negativer Innenoberfläche unter Druck und bei erhöhter Temperatur aufgeschäumten harten Polyadditionsprodukt von Polyol und Isocyanat, (polyurethan) mit geschlossener Zellenstruktur, von hohem Molgewicht und hohem Vernetzungsgrad.

Es wird in der Form gebildet durch Mischen von nahezu gleichen Gewichtsteilen Polyoxypropylenglykol und Toluoldiisocyanat, wobei dem Gemisch 0.2 Gew.-% Triäthylendiamin und 0.7 Gew.-% Kohlendioxyderzeugendes Schäummittel zugegeben wurde.

Man lässt das Gemisch bei 75° C in der Form reagieren und expandieren und danach unter Ueberdruck von 150 bar durchhärten.

Die Aussparungen für die Halterungen der Gasentladungsröhren, die Leitungen, die Schaltteile und die Lagerungsbuchsen für die Profile, die die Kippstativvorrichtung bilden, werden bereits durch die gestaltende Form erzeugt.

Hohlräume Können auch durch fräsen, bohren oder sägen im Material erzielt werden.

Die nachfolgende Tabelle II gibt die Werte, die typisch sind für die Bildung des Polyurethankunststoffs:

Tabelle II:

Mischverhältnis Polyol/isocyanat (Gew.tle) 110:100.

| Isocyanat-Index: | 9 |
|---|---|
| Standzeit: | 31 sek. |
| Fadenziehzeit: | 91 sek. |
| Klebrigkeitszeit: | 113 sek. |
| Steigzeit: | 150 sek. |
| Dichte: | 80.5 g/l. |

Die Erfindung wird weiter erläutert an Hand der beigefügten Figuren, die schematisch Abbildungen und Querschnitte der Besonnungskombination, wie sie erfindungsgemäss gefertigt wurde, darstellen.

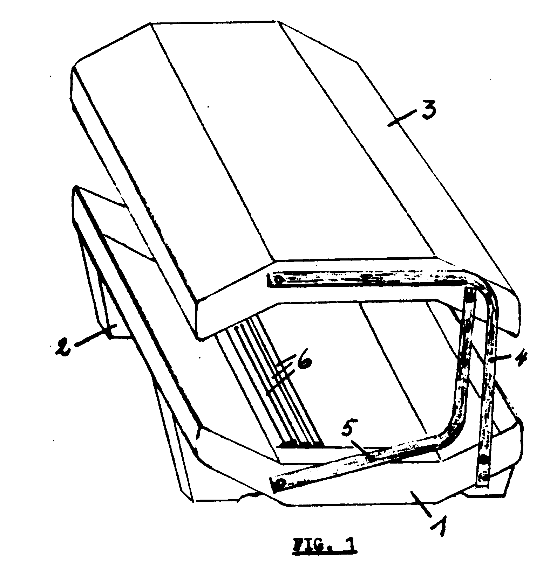

Figur 1 stellt eine isometrische Abbildung der Kombination dar.

Darin ist (1) die Liege (Unterteil, Wanne) mit den nach oben gebogenen langen Seitenkanten und den Füssen (2); der Oberteil (Sonnenkuppel) ist (3), und die beiden Profile (4) und (5) halten den Oberteil in jeder gewünschten Stellung.

Hier ist die tiefste Stellung, parallel zur Liege, gezeichnet.

Die Röhren sind mit (6) angegeben.

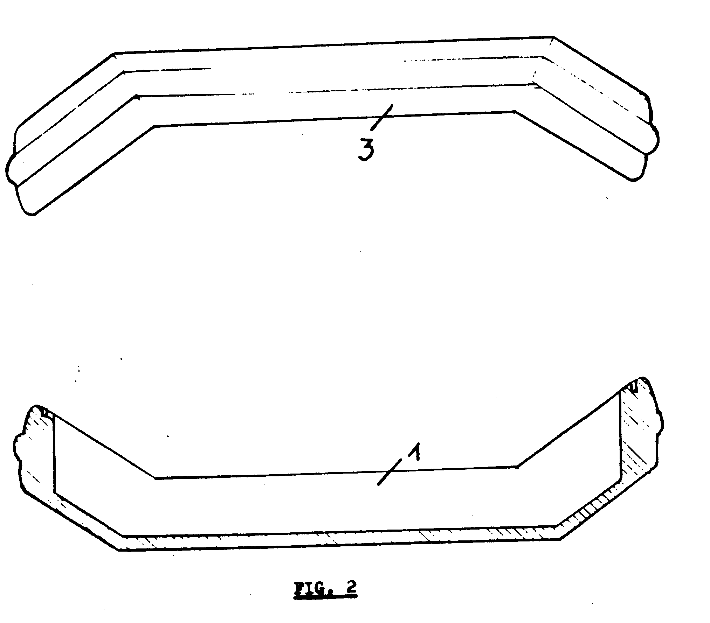

Figur 2 zeigt einen senkrechten Querschnitt durch die Besonnungsliege mit der Sonnenkuppel darüber.

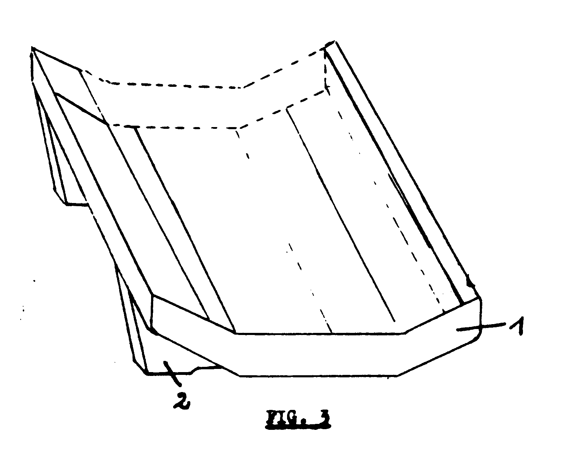

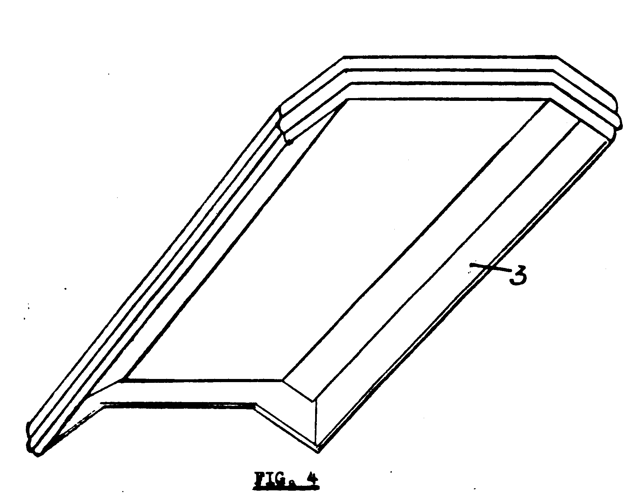

Figur 3 zeigt eine isometrische Abbildung der Besonnungsliege ohne die Halterungsprofile, und Figur 4 zeigt eine Abbildung des Oberteils von unten gesehen.

**Patentansprüche**

1. Besonnungskombination, bestehend aus einer Liege, deren beide Längskanten in einem Winkel von etwa 30° aufwärts gebogen sind, und einem Oberteil (Sonnenkuppel), deren beide Längskanten in einem Winkel von etwa 30° abwärts gebogen sind, wobei beide Teile je etwa 10 UV-Gasentladungsröhren enthalten, die sich über die Gesamtlänge erstrecken, dadurch gekennzeichnet, dass jedes der beiden Teile der Kombination aus einem nahtlosen Ganzen von aufgeschäumtem Hartpolyurethan besteht, in welchem alle erforderlichen Halterungs-, Leitungs- und Schaltteile doppelt isoliert eingebettet sind, und wobei die beiden genannten Teile an den Endkanten von Kopf-und Fussende jeweils miteinander verbunden sind durch eine Kippstativvorrichtung, die aus zwei zu einem Winkel gebogenen

3

Profilen besteht, und deren Enden in drehbarer Lagerung jeweils die Endkanten des Liegeteils und des Oberteils erfassen, sodass einzig durch diese beiden Profile eine stabile Stellung in jeder gewünschten Position erreicht wird, und der Oberteil von Hand anstrengungslos auf-und abwärts bewegt werden kann.

2. Besonnungskombination nach Anspruch 1, dadurch gekennzeichnet, dass die Körper der Leige und des Oberteils hergestellt sind aus einem Stück nahtlos in einer Form mit negativer Innenoberfläche unter Druck und bei erhöhter Temperatur aufgeschäumtem harten Polyadditionsprodukt von Polyolen und Isocyanat (Polyurethan) mit geschlossener Zellenstruktur von hohem Molgewicht und hohem Vernetzungsgrad.

3. Verfahren zur Herstellung einer Besonnungskombination, bestehend aus einer Liege und einem darüber angebrachten Oberteil (Sonnenkuppel), in deren jeder UV-Gasentladungsröhren über die gesamte Länge gelegt werden, dadurch gekennzeichnet, dass man die Körper der Liege und des Oberteils jeweils in einer Form gestaltet durch Mischen von nahezu gleichen Gewichtsteilen Polyoxypropylenglykol und Toluoldiisocyanat, welchem Gemisch 0.2 Gew.-% Triäthylendiamin und 0.7 Gew.-% Kohlendioxyd-entwickelndes Schaummittel zugefügt werden, man das Gemisch bei 75°C in der Form reagieren und expandieren lässt und danach unter Ueberdruck von 150 bar durchhärten lässt, wonach jeweils an den Endkanten die Enden von zwei zu einem Winkel gebogenen Profilen in drehbarer Lagerung angebracht werden.

## Claims

1. Sun lamp appliance consisting of a reclining settee with both rims bent upwards at an angle of about 30° and an upper part (sun canopy) with both rims bent downwards at an angle of about 30°, each of said parts comprising about 10 tubular gas discharge UV-lamps, extending over the total length of same, characterized in that each of said parts consists of a seamless body of expanded rigid polyurethane, in which all necessary bearing parts, connections and switching parts are embedded in a double insulated manner, and whereat both said parts are connected at their head as well as at their foot ends by means of an overhead tilting frame, consisting of two profile sections bent at an angle, the ends of which keeping the end girders of the settee and of the canopy in a turnable bearing, such that only by means of said two profile sections a stable fixation in any desired position can be obtained, and the canopy can be moved upwards and downwards without any exertion by hand.

2. Sun lamp appliance according to claim 1, characterized in that the bodies of the settee and of the upper part are manufactured of a piece of rigid reaction product of polyaddition of polyols and isocyanate (polyurethane) of high molecular weight and high degree of cross-linking, having a seamless structure of closed cells, expanded under pressure and under increased temperature in a mould with a negative inner surface.

3. A process for the manufacture of a sun lamp appliance, consisting of a reclining settee and an upper part (sun canopy) situated across, in each of which tubular gas discharge UV-lamps are laid over the total length, characterized in that the bodies of said settee and of said upper part are shaped in a mould by mixing of about equal parts of weight of polyoxypropylene-glycol and toluene-di-isocyamate, with adding to this mixture 0.2% by weight of triethylenediamine and 0.7% by weight of carbon-dioxide-developing foaming agent, the mixture reacting and expanding at 75°C and further curing at an overpressure of 150 bar, whereafter at each of the end rims the ends of two profile sections bent at an angle are attached in a turnable bearing.

## Revendications

1. Un appareillage à l'exposition aux lampes de soleil subsistant d'une couche, dont les deux côtés latéraux sont courbés dans un angle de 30° environ vers le haut, et d'une part supérieure (comme ciel solaire), dont les deux côtés latéraux sont courbés vers le bas dans un angle de 30°, chacune des deux parties comportant d'environ dix lampes tubulaires émanant un rayonnement de lumière ultraviolet, s'étendant le long de la longueur totale, caractérisé en ce que chacune desdites parties consiste d'un ensemble sans sutures de polyuréthane expandé et rigide, dans lequel toutes les parts essentielles pour la stabilité, les lignes électriques et les connexions sont abritées d'une manière d'isolation

4

double, et auquel lesdites parties sont combinées l'une à l'autre au chevet et au pied par un support inclinable, consistant en deux profils courbés dans un angle, et dont les extrémités tiennent les côtés aboutissants de la couche et de la part supérieure à roulement rotatif, de sorte qu'on puisse gagner une position stable en chaque position désirée seulement au moyen de ces deux profils, et qu'on puisse mettre en mouvement la part supérieure en haut et en bas à la main sans faire des efforts.

2. Un appareillage aux lampes de soleil d'après la revendication 1, caractérisé en ce que les ensembles de la couche et de la part supérieure sont fabriqués d'une pièce rigide sans sutures d'un produit de réaction de polyaddition de polyoles et d'isocyanate (polyurethane) avec une structure de cellules fermées et d'un poids moléculaire élevé et d'un degré de réticulation considérable, obtenu dans une forme avec une surface negative sous pression et température elevée.

3. Un procédé de fabrication d'un appareillage aux lampes de soleil, subsistant d'une couche et d'une part supérieure (comme ciel solaire) située tout en haut, dans lesquelles des lampes tubulaires émanant un rayonnement ultraviolet sont posées dans la longueur totale, caractérisé en ce que les ensembles de la couche et de la partie supérieure sont fabriqués dans une moule en mélangeant de parts en poids égalées de polyoxypropylène glycol et de toluène di-isocyanate, dans lequel on ajoute 0.2% en poids de triéthylène-diamine et de 0.7% en poids d'agent gonflant dégageant le dioxide de carbone, puis le mélange réagit, expand à 75°C et durcit sous pression de 150 bar et enfin aux côtés des extrémités les bords de deux profils courbés sont insérés sous forme d'un angle dans un roulage rotatif.

FIG. 1

3

1

**FIG. 2**

FIG. 3

FIG. 4